Europäisches Patentamt

European Patent Office

Office européen des brevets

(19)

(11) Numéro de publication: **0 421 876 A1**

## DEMANDE DE BREVET EUROPEEN

(12)

(21) Numéro de dépôt: 90402746.3

(51) Int. Cl.⁵: **C12P 21/08**

(22) Date de dépôt: 04.10.90

(30) Priorité: 06.10.89 FR 8913074

(43) Date de publication de la demande:
10.04.91 Bulletin 91/15

(84) Etats contractants désignés:
**BE DE GB IT NL**

(71) Demandeur: **PASTEUR MERIEUX SERUMS ET VACCINS, Sociéte Anonyme :**
**58 Avenue Leclerc**
**F-69348 Lyon Cédex 07(FR)**

(72) Inventeur: **Carosella, Edgardo D.**
**27 rue Soeurs Bouvier**
**F-69005 Lyon(FR)**
Inventeur: **Latour Née Panero, Mireille**
**23 Les Sabines**
**F-69130 Ecully(FR)**
Inventeur: **Armand, Jacques**
**Le Clos, St. Germain S/I'Arbresle**
**F-69210 L'Arbresle(FR)**
Inventeur: **Mawas, Claude**
**8 rue des Cinq Cents**
**F-13007 Marseille(FR)**
Inventeur: **Olive, Daniel**
**56 avenue Massenet**
**F-13009 Marseille(FR)**
Inventeur: **Emilie, Dominique**
**15 rue Auguste Lançon**
**F-75013 Paris(FR)**

(74) Mandataire: **Bernasconi, Jean et al**
**CABINET LEMOINE ET BERNASCONI 13,**
**Boulevard des Batignolles**
**F-75008 Paris(FR)**

(54) Application d'agents actifs pour la préparation d'un médicament destiné au traitement de lymphomes ou assimilés.

(57) Les anticorps capables de se fixer sur le récepteur de l'interleukine-2 humaine sont appliqués à la préparation d'un médicament destiné au traitement des lymphomes et de tumeurs similaires à cellules tumorales qui impliquent l'expression du récepteur de l'interleukine-2.

EP 0 421 876 A1

L'invention concerne l'application d'agents actifs pour la préparation d'un médicament destiné au traitement de lymphomes ou assimilés chez l'homme.

Les affections cancéreuses du tissu lymphoïde ou des lymphocytes, telles que les lymphomes à lymphocytes T ou B, etc., se traduisent notamment par une prolifération des lymphocytes concernés formant des masses tumorales solides.

Les traitements mis en oeuvre pour ces affections diffèrent beaucoup les uns des autres, selon qu'il s'agit de leucémies ou de tumeurs solides et selon les différents types de maladies au sein d'une même catégorie. Les résultats sont insuffisants et le pronostic négatif dans de nombreux cas.

T.A. WALDMANN et al., dans Blood (1988) 72, 1805-1816 ont montré, chez 3 patients sur 9, l'intérêt d'un anticorps monoclonal de souris anti-Tac, reconnaissant le récepteur de l'interleukine-2, (CD25), dans le cas d'une leucémie à lymphocytes T de l'adulte associée à HTLV-1. Cet anticorps se fixe sur le récepteur de l'interleukine-2 et inhibe la prolifération des lymphocytes normalement induite par l'interleukine-2.

D. OLIVE et al., (Eur. J. Immunol., 1986, 16:611-616) ont décrit un panel d'anticorps monoclonaux dirigés contre différents épitopes de la chaîne α (p 55) du récepteur de l'interleukine-2 humaine.

La déposante a trouvé que des anticorps monoclonaux reconnaissant le récepteur de l'interleukine-2 humaine sont efficaces dans le traitement des lymphomes et de tumeurs similaires à cellules tumorales qui impliquent l'expression du récepteur de l'interleukine-2 humaine par les lymphocytes T et par certains lymphocytes B.

L'invention a donc pour objectif de fournir des médicaments à base d'anticorps qui soient efficaces dans leur utilisation pour le traitement des lymphomes et de tumeurs similaires à cellules tumorales cités ci-dessus.

Un autre objectif de l'invention est de fournir de tels médicaments qui soient peu ou pas immunogènes.

L'invention a donc pour objet l'application d'anticorps capables de se fixer sur le récepteur de l'interleukine-2 humaine, pour la préparation d'un médicament destiné au traitement des lymphomes et de tumeurs similaires à cellules tumorales qui impliquent l'expression du récepteur de l'interleukine-2.

Selon un mode de réalisation particulier de l'invention, l'anticorps capable de se fixer sur le récepteur de l'interleukine-2 humaine est un anticorps monoclonal (AcM) de rat, notamment un anticorps de classe IgG2a et en particulier l'anticorps 33B3.1 (selon D. OLIVE et al., Eur. J. Immunol. 1986, 16:611-616).

Selon un autre mode de réalisation, l'anticorps capable de se fixer au récepteur de l'interleukine-2 humaine est un anticorps "humanisé" comprenant une région d'origine animale, qui reconnaît le récepteur de l'interleukine-2, et une région d'origine humaine.

De préférence, la région d'origine animale est un fragment Fab ou F(ab')$_2$ d'un anticorps monoclonal de rat, notamment de l'anticorps 33B3.1.

Les fragments Fab et F(ab')$_2$ peuvent être des fragments d'anticorps monoclonaux obtenus par immunisation d'animaux contre le récepteur de l'interleukine-2 humaine (RIL2).

De préférence, la région d'origine humaine est une IgG humaine ou un fragment Fc d'une IgG humaine.

Ces fragments Fc peuvent également être obtenus par coupure enzymatique sélective d'une IgG humaine, selon la méthode décrite par Barandun S. et al., Vox Sang. 28, 157 (1975). Dans ce cas, la région d'origine animale est un fragment Fab.

Cette région d'origine humaine peut être un anticorps polyclonal ou monoclonal.

De préférence, lesdites IgG ou lesdits fragments Fc sont respectivement des isotypes IgG 1, 2, 3 ou 4. Les fragments Fc peuvent aussi être obtenus par génie génétique.

Les anticorps anti-RIL2 de l'invention peuvent également être construits par expression d'ADN recombinant, selon les méthodes du génie génétique, par exemple selon la méthode de Zenon Steplewski et al., Proc. Natl. Acad. Sci. USA, Vol. 85, 4852-4856, juillet 1988.

L'invention a également pour objet des médicaments comprenant les anticorps selon l'invention et des procédés de traitement des patients souffrant de lymphomes ou tumeurs similaires solides, selon l'invention, lesdits procédés utilisant les médicaments selon l'invention.

Les médicaments selon l'invention comportent les anticorps anti-RIL2 définis ci-dessus, de préférence sous forme lyophilisée ou sous forme de solution pour administration parentérale, sous une dose efficace.

L'invention va être maintenant décrite plus en détail à l'aide d'un exemple de procédé de préparation d'anticorps selon l'invention et d'un exemple non limitatif d'application d'un anticorps selon l'invention à la réalisation d'un médicament destiné à un traitement thérapeutique.

I - Préparation d'anticorps humanisés

1) Anticorps Fab-IgG

a) Obtention d'IgG humaines réduites

Le produit de départ est une solution à 50 mg/ml d'IgG polyclonales humaines en tampon 0,2M Tris HCl pH 8,0 contenant 20mM de dithiothreitol et 1mM d'éthylènediaminetétracétate disodique (Na$_2$ EDTA). Après 30 minutes d'incubation à 30°C, on obtient une solution d'IgG humaines réduites. On purifie la solution obtenue par dialyse contre un tampon acétate de sodium 0,5 M 10mM éthylènediaminetétracétate disodique (pH 5,4).

On obtient ainsi une solution purifiée d'IgG humaines réduites en tampon acétate.

b) Obtention de fragments Fab d'un anticorps monoclonal de rat.

L'anticorps monoclonal de départ est un anticorps de rat anti-RIL2 humain d'isotype IgG2a (référence 33B3.1) sous la forme d'une solution dans une solution saline isotonique.

On purifie la solution par dialyse pendant 16 heures contre une solution de formiate de sodium 0,1M pH 2,8, puis par dialyse pendant 24 heures contre une solution d'acétate de sodium 0,1M pH 4,5.

On ajoute ensuite une solution de pepsine jusqu'à une concentration de 2%, pH 4,5 et on laisse incuber pendant 4 heures à 37°C. On arrête alors la réaction enzymatique par remontée du pH à 8.

Par chromatographie de perméation sur Séphacryl S 200 (Pharmacia) et élution avec un tampon Tris 0,2M pH 8,0, on obtient une solution à 5 mg/ml de fragments F(ab')$_2$. On ajoute du bêta-mercapto-éthanol jusqu'à une concentration de 10mM et on laisse incuber pendant 30 minutes à 30°C.

Par chromatographie sur Sephadex G 25 (Pharmacia), et élution par un tampon acétate de sodium 50mM + 2mM Na$_2$ EDTA, pH 5,3 on obtient une solution de fragments Fab' exempte de bêta-mercapto-éthanol.

L'anticorps monoclonal de départ a été décrit par D. OLIVE et al., article cité ci-dessus.

c) Préparation de conjugué Fab-orthophénylènedimaléimide.

L'orthophénylènedimaléimide en solution 4mM, obtenue par dissolution de 18,76 mg dans 3,5 ml de diméthylformamide, est ajouté à la solution de fragments Fab.

On laisse incuber pendant 45 minutes à 4°C sous agitation, à pH 4,7.

La solution est ensuite chromatographiée sur Phospho Ultragel AGR (IBF) équilibré avec un tampon acétate de sodium 40mM, Na$_2$ EDTA 2mM, pH 4,7. On élue avec un tampon acétate de sodium 800mM, Na$_2$ EDTA 5mM, pH 5,7. On obtient une solution de fragments Fab maléimidés.

d) Couplage du fragment Fab avec l'IgG réduite.

On mélange la solution obtenue au stade c) et la solution d'IgG humaines réduites obtenues au stade a) ; rapport molaire 1/1.

On laisse incuber pendant 24 heures à 4°C puis on soumet à une dialyse contre un tampon PBS pendant 16 heures environ.

Par chromatographie (Sephacryl S 200 ou S 300 (Pharmacia)), on sépare une solution de la chimère Fab-IgG obtenue.

En électrophorèse en S.D.S. PAGE, la chimère Fab-IgG est caractérisée par une bande dont le poids moléculaire est environ 230 KD.

2) Préparation d'anticorps chimériques du type Fc-Fab et Fc-2Fab.

a) De façon analogue à celle décrite à l'exemple 1, stade a, en remplaçant les IgG par des fragments Fc d'IgG humaines, on obtient une solution purifiée de fragments Fc réduits.

3

Les fragments Fc utilisés comme produits de départ sont obtenus à partir d'IgG polyclonales humaines selon la méthode de Barandun S. et al., Vox Sang. 28, 157 (1975).

b) Les fragments Fab sont obtenus comme à l'exemple 1 stade b, et couplés à l'orthophénylènedimaléimide comme à l'exemple 1, stade c.

c) On mélange la solution de fragments Fab couplés au dimaléimide, obtenue au stade b, avec la solution de fragments Fc réduits obtenue au stade a (rapport molaire : 2:1).

On laisse en contact 24 heures à +4°C puis on met en dialyse contre du PBS à +4°C.

A la sortie de dialyse, on réalise une chromatographie sur Sephacryl S 200 (filtration sur gel) en colonne de 1m de longueur.

On réalise trois chromatographies successives pour séparer les chimères puis une reconcentration par ultrafiltration. On obtient ainsi une chimère du type Fc-2Fab et une chimère du type Fc-Fab, caractérisées en électrophorèse et en filtration sur gel.

En électrophorèse (SDS-PAGE), les deux chimères ont respectivement des masses molaires de 160 000 et 100 000 environ.

## 3) Présentation

Le médicament préparé selon l'invention est présenté en flacons, à l'état lyophilisé. Il est à reconstituer par 10 ml d'eau pour l'injection par perfusion intraveineuse après dilution dans 50 à 100 ml de soluté physiologique.

La composition du lyophilisat est la suivante :

| IgG2a | 10 mg |
|---|---|
| NaCl | 80 mg |
| Mannitol | 100 mg |
| $Na_2HPO_4$, $2H_2O$ | 22,8 mg |
| $NaH_2PO_4$, $2H_2O$ | 11,7 mg |

## II - Application

On s'est penché sur le cas d'un patient souffrant d'un lymphome à cellules géantes anaplastiques (ALCL) exprimant Ki-1 et CD25 (Ki-1 = antigène de membrane épithéliale ; CD25 = récepteur de l'interleukine-2 - G. DELSON et al., Amer. J. Pathol. (1988) 130, 59-70) et pour lequel plusieurs chimiothérapies avaient échoué.

Le patient, un homme de 64 ans, de type caucasien, présentait en octobre 1988 une infiltration multinodulaire de la peau de l'aine gauche et des hypertrophies du foie et des ganglions lymphatiques axillaires, inguinaux et cervicaux. Des lymphadénopathies médiastinales et abdominales et des infiltrations nodulaires des poumons apparaissaient en scintillographie CT thoraco-abdominale.

L'examen cytologique et histologique d'une biopsie du ganglion lymphatique correspondait à un ALCL (STANSFELD, A.G. et al., J.A.M. & Wright, D.H. Updated Kiel classification for lymphomas - Lancet i, 1988, 192-193 ; N.C.I. non Hodgkin's classification project writing committee - Classification of non Hodgkin's lymphomas. Reproductibility of major classification systems. Cancer (1985) 55, 91-95).

Ce diagnostic a été confirmé par des études immunohistochimiques. Les cellules lymphomateuses ont été observées sur une ponction de moelle osseuse et sur une biopsie de peau. Des hybridations in situ ont été effectuées sur les ganglions lymphatiques et sur des zones cutanées du lymphome malin à l'aide de sondes spécifiques IL-2 (Interleukine-2) et ont montré la présence de cellules productrices d'ARNm d'IL-2. Les analyses sérologiques étaient négatives pour les anticorps anti-HIV-1 et anti-HTLV-1.

Le patient a été traité par différentes chimiothérapies, mais la tumeur a régulièrement et rapidement progressé.

On a ensuite tenté un traitement mettant en oeuvre un anticorps monoclonal (AcM) anti-CD25, à savoir le 33B3.1 (D. OLIVE et al. cité ci-dessus et J.P. SOUILLOU et al., Lancet (1987) i , 1339-1342).

On a administré 33B3.1 par voie intraveineuse à raison de 10 mg par jour avec, simultanément, 40 mg de méthylprednisolone et 50 mg de cyclophosphamide, l'objectif étant de diminuer la réponse anticorps contre 33B3.1.

On a constaté une réponse clinique à partir du cinquième jour de traitement et jusqu'au vingtième jour :
- régression de la tumeur (scintillographie CT pulmonaire) ;
- nécrose de tous les nodules de la peau ;
- forte diminution de taille des ganglions lymphatiques ;
- disparition des oedèmes ainsi que de la paralysie faciale ;
- pas de cellules malignes au dix-huitième jour (ponction de moelle osseuse) ;
- le niveau du sérum en lacticodéshydrogénase (LDH) est passé de 629 U/ml à 326 U/ml (normal < 260 U/ml).

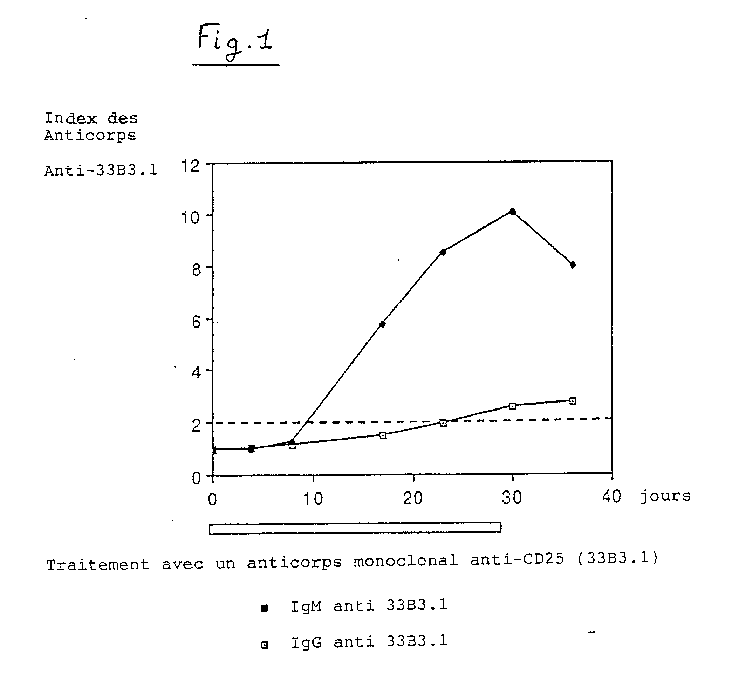

La figure 1 présente l'évolution du taux d'anticorps anti-33B3.1 au cours du temps de traitement. Une réponse immunitaire anti-33B3.1 est apparue au cours du traitement, des IgM anti-33B3.1 ayant été détectées au seizième jour du traitement et des IgG anti-33B3.1, au vingt-troisième jour.

On a arrêté le traitement aux AcM anti-CD25 le vingt-huitième jour. Malgré la reprise de la chimiothérapie, le lymphome s'est remis à progresser.

Les anticorps monoclonaux anti-CD25 hétérologues peuvent donc être utilisés dans le traitement des lyphomes ou des tumeurs similaires exprimant le récepteur pour l'IL-2 (CD-25), notamment dans le but de potentialiser les effets d'une chimiothérapie conventionnelle. En raison de l'absence d'effets secondaires constatés, un traitement suivi peut être mis en oeuvre à l'aide de médicaments à base d'anticorps "humanisés" décrits dans la présente demande.

La souche productrice d'anticorps 33B3.1, disponible (voir la citation D. OLIVE et al., précitée), a fait l'objet d'un dépôt auprès de la Collection Nationale de Cultures de Microorganismes (CNCM) sous le n° I-1002.

## Revendications

1. Application d'anticorps capables de se fixer sur le récepteur de l'interleukine-2 humaine, pour la préparation d'un médicament destiné au traitement des lymphomes et de tumeurs similaires à cellules tumorales qui impliquent l'expression du récepteur de l'interleukine-2.

2. Application selon la revendication 1, caractérisée en ce que l'anticorps capable de se fixer sur le récepteur de l'interleukine-2 humaine est un anticorps monoclonal de rat.

3. Application selon la revendication 2, caractérisée en ce que l'anticorps monoclonal de rat est un anticorps de classe IgG2a:

4. Application selon la revendication 3, caractérisée en ce que l'anticorps monoclonal de rat est l'anticorps 33B3.1.

5. Application selon la revendication 1, caractérisée en ce que l'anticorps capable de se fixer sur le récepteur de l'interleukine-2 est un anticorps humanisé comprenant une région d'origine animale, qui reconnaît le récepteur de l'interleukine-2, et une région d'origine humaine.

6. Application selon la revendication 5, caractérisée en ce que la région d'origine animale est un fragment Fab d'un anticorps monoclonal de rat.

7. Application selon l'une des revendications 5 et 6, caractérisée en ce que la région d'origine animale provient d'un anticorps monoclonal 33B3.1.

8. Application selon l'une des revendications 5 à 7, caractérisée en ce que la région d'origine humaine est une IgG humaine.

9. Application selon la revendication 5, caractérisée en ce que la région d'origine humaine est un anticorps monoclonal ou polyclonal.

10. Application selon la revendication 9, caractérisée en ce que lesdites IgG ou lesdits fragments Fc sont respectivement des isotypes IgG1, 2, 3 ou 4.

11. Application selon l'une quelconque des revendications 1 à 8, caractérisée par le fait que l'anticorps anti-RIL2 humain est obtenu par expression d'ADN recombinant.

## *Fig.1*

Index des
Anticorps

Anti-33B3.1

Traitement avec un anticorps monoclonal anti-CD25 (33B3.1)

■    IgM anti 33B3.1

▫    IgG anti 33B3.1

**Office européen
des brevets**

**RAPPORT DE RECHERCHE
EUROPEENNE**

Numéro de la demande

**EP 90 40 2746**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,P | WO-A-8 909 622 (PROTEIN DESIGN LABS) <br> * Page 12, lignes 10-14 * <br> — — — | 1-11 | C 12 P 21/08 |
| X | EP-A-0 296 082 (IMMUNOTECH) <br> * Page 2, ligne 1 - page 3, ligne 2 * <br> — — — | 1-11 | |
| X | EP-A-0 241 811 (BAYER) <br> * Page 1, lignes 1-7 * <br> — — — | 1-11 | |
| X,D | BLOOD, vol. 72, no. 5, novembre 1988, pages 1805-1816, Grune <br> & Stratton, Inc.; T.A. WALDMANN et al.: "Therapy of patients with human T-cell lymphotrophic virus I-induced adult T-cell leukemia with anti-tac, a monoclonal antibody to the receptor for interleukin-2" <br> — — — — — | 1-11 | |

**DOMAINES TECHNIQUES
RECHERCHES (Int. Cl.5)**

C 12 P

Le présent rapport de recherche a été établi pour toutes les revendications

| Lieu de la recherche | Date d'achèvement de la recherche | Examinateur |
|---|---|---|
| La Haye | 15 novembre 90 | TURMO Y BLANCO C.E. |